# EUROPEAN PATENT SPECIFICATION

(11) **EP 0 334 455 B1**
(45) Date of publication and mention of the grant of the patent: **16.06.1993**
(21) Application number: 89200728.7
(22) Date of filing: 21.03.1989
(51) Int. Cl.: C07C 69/716, C07C 67/347

(54) **Process for the production of keto-diesters**
Verfahren zur Herstellung von Ketodiestern
Procédé de préparation de diesters cétoniques

(30) Priority: 23.03.1988 US 171999
(43) Date of publication of application: 27.09.1989
(73) Proprietor: SHELL INTERNATIONALE RESEARCH MAATSCHAPPIJ B.V., 2596 HR Den Haag (NL)
(72) Inventor: Wang, Pen Chung, Houston Texas 77079 (US)

(56) References cited:
- US-A- 2 637 742
- CHEMISCHE BERICHTE, vol. 114, 1981, pages 1226-1233, Verlag Chemie, GmbH, Weinheim, DE; H. STETTER et al.: "Neue Methode zur Darstellung symmetrischer 1,4-Diketone"
- CHEMISCHE BERICHTE, vol. 113, 1980, pages 690-698, Verlag Chemie, GmbH, DE; H. STETTER et al.: "Addition von aliphatischen Aldehyden an alpha,beta-ungesättigte Carbonsäureester und Nitrile"
- CHEMISCHE BERICHTE, vol. 109, 1976, pages 3426-3431, Verlag Chemie, GmbH, Weinheim, DE; H. STETTER et al.: "Addition aliphatischer, heterocyclischer und aromatischer Aldehyde an Butenon"

## Description

This invention relates to a process for the production of keto-diesters. More particularly, the invention relates to the production of certain keto-diesters by reacting an alkyl ester of a 2-alkenoic acid with formaldehyde in the presence of a catalyst.

Reaction of an aldehyde and unsaturated or acidic compounds in the presence of an initiator or catalyst is broadly old in the art with the type of end product depending upon the particular chemical nature of the reactants and the type of catalyst or initiator employed.

Generally those processes are useful in increasing the functionality of the unsaturated or acidic reactant through the formation of 1:1 addition products. However, it would be useful to provide a reaction of unsaturated carboxylic acid esters and aldehydes wherein the condensation is in a ratio of greater than 1:1, i.e. 2:1, and thereby produce functionally substituted products of increased functionality. It is already known (e.g. from US Patent 2637742 and Chem. Ber. 113, 690-698 (1980)) to react certain aldehydes with acrylates in the presence of a free-radical promoting catalyst (e.g. thiazolium salts containing trialkylamine as promoter, as described in Chem. Ber. 113, 690-698 (1980)). However, the aldehydes hitherto used contain only one formylic hydrogen atom (the term "formylic hydrogen atom" refers to a H-atom directly bound to the C-atom of a C=O-group), and keto-diesters are not formed by the reaction. It has now been found that the use of formaldehyde, which contains two formylic hydrogen atoms, enables keto-diesters to be produced.

Accordingly, the invention relates to a process for the preparation of a keto-diester of formula:- 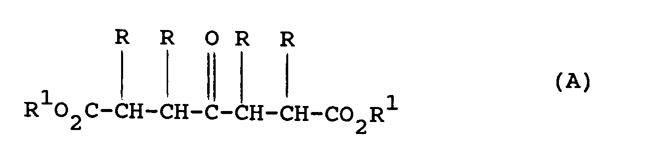
wherein each R independently represents a hydrogen atom or a methyl group, and each R¹ represents an alkyl group of up to 4 carbon atoms, which comprises reacting formaldehyde with an alkyl ester of a 2-alkenoic acid of the formula: 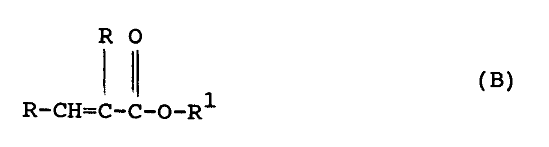
wherein R and R¹ have the same meanings as for formula (A), the reaction being carried out in the liquid phase and in the presence, as catalyst, of a thiazolium salt.

The lower alkyl esters of acrylic acid and methacrylic acid are preferred, especially methyl acrylate. The formaldehyde reactant may be provided in any of the conventional forms including trioxane and paraformaldehyde.

The precise ratio of alkenoic acid ester to formaldehyde to be employed is not critical although a molar excess of ester is desired because of the 2:1 stoichiometry of the reaction. Molar ratios of alkenoic acid ester of aldehyde from 1:1 to 5:1 are satisfactory with molar ratios from 1.5:1 to 3:1 being preferred.

The catalyst employed in the process of the invention preferably is a thiazolium salt. Without wishing to be bound by any particular theory, it is considered likely that the thiazolium moiety undergoes transitory participation through the 2-position and a hydrogen substituent on that 2-position. The substitution on the 4- and 5- carbon atoms of the thiazolium salt is not critical so long as the substituents are inert to the reactants and the condensation product thereof. The precise nature of the salt is also not critical although thiazolium halides are generally preferred thiazolium salts, particularly the middle halides, chlorides and bromides, and best results are obtained when a thiazolium chloride is utilized as catalyst. A typical and conventional thiazolium salt production involves the reaction of a thiazole and an alkyl, including arylalkyl, chloride. Formation of the salt serves to introduce the alkyl group of the alkyl chloride on the 3-position of the thiazole.

The preferred thiazoium salts are represented by the formula 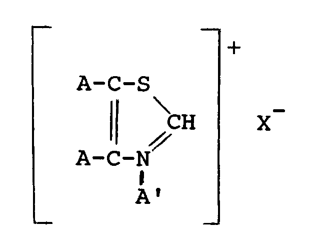
wherein X is middle halogen, preferably chlorine, A independently is hydrogen, alkyl of up to 8 carbon atoms, aryl of up to 8 carbon atoms or 2-hydroxyethyl, and A' is alkyl including arylalkyl of up to 8 carbon atoms.

Illustrative of suitable thiazole precursors of the thiazolium salt catalysts of the invention are thiazole, 4,5-dimethylthiazole, 4-methyl-5-(2-hydroxyethyl)thiazole, 4-phenylthiazole, 5-methyl-thiazole and 4-methyl-5-benzylthiazole. Corresponding thiazolium salts which are suitably employed as the catalyst in the process of the invention include thiazolium hydrochloride, 3-benzyl-4,5-dimethyl-thiazolium chloride, 3-benzyl-4-methyl-5-(2-hydroxy-ethyl)thiazolium chloride and 3,5-dibenzyl-4-methyl-thiazolium chloride. Such thiazolium salts are conventional and some are commercially available. Particularly preferred as the thiazolium salt is 3-benzyl-4-methyl-5-(2-hydroxyethyl)thiazolium chloride.

The thiazolium salt is employed in catalytic amounts, i.e., amounts which are less than stoichiometric with the ester and aldehyde reactants. Suitable amounts of thiazolium salt catalyst are from 0.01 mole to 0.5 mole per mole of thiazolium salt per mole of the aldehyde reactant, preferably from 0.05 mole to 0.3 mole of thiazolium salt per mole of aldehyde.

The catalyst suitably contains a promoter, in particular a trialkylamine, preferably at least two alkyl has up to 12 carbon atoms inclusive. Preferably at least two alkyls are lower alkyl, i.e. contain up to four carbon atoms. Illustrative of such trialkylamines are trimethylamine, triethylamine, trioctylamine, dimethyldodecylamine, dimethylethylamine, diisopropylbutylamine and methylethylpropylamine Largely for convenience, the preferred trialkylamines are trimethylamine and triethylamine, particularly triethylamine The amine is employed in quantities which are approximately equimolar with the unsaturated ester. Quantities of tertiary amine catalyst promoter from 0.7 mole to 1.6 mole per mole of unsaturated ester reactant are satisfactory with quantities of amine from 0.8 mole to 1.2 mole per mole of ester reactant being preferred.

The condensation reaction of the invention is conducted in liquid phase solution in an inert reaction environment. Suitable as the reaction solvent are the lower alkanols such as methanol and ethanol or aromatic hydrocarbon solvents such as toluene or xylene. The reactants are contacted in the presence of the catalyst and catalyst promoter by conventional methods such as stirring, shaking or refluxing. The use of an elevated reaction temperature is advantageous, and reaction temperatures from 35°C to 120°C are suitable with temperatures from 40°C to 100°C being preferred. The reaction pressure to be employed is at least sufficient to maintain the reaction mixture in the liquid phase. Typical reaction pressures are from 0.8 bar to 10 bar, more typically from 1 bar to 3 bar. Subsequent to reaction the product mixture is separated and the desired keto-diester recovered by conventional methods such as distillation and selective extraction.

The keto-diester product of the invention is a 2:1 condensation product of the 2-alkenoic acid ester and formaldehyde. By way of illustration, when the ester reactant is methyl acrylate, each R of the formula (A) given earlier will be hydrogen, and R¹ will be methyl. The resulting product is dimethyl 4-oxoheptanedioate.

The products of the invention are polyfunctional α,ω-diesters having ketone functionality in the central portion of the molecule and accordingly are useful as chemical intermediates for a number of applications. Particular applications include reaction with diamines to produce polyamides and reaction with diols to produce polyesters. Such polyamides or polyesters are thermoplastic polymers which are processed by conventional methods into sheets, films and molded articles having utility, inter alia, as packaging materials.

The invention is further illustrated by the following Example.

### EXAMPLE 1

A solution of paraformaldehyde (9.0g, 0.3 mole), 3-benzyl-5-(2-hydroxethyl)-4-methylthiazolium chloride (16.0g, 0.6 mole), triethylamine (36.0g, 0.6 mole) and methyl acrylate (50.0g, 0.58 mole) in 500 ml of ethanol was heated under nitrogen at 60-70°C for 24 hours. The solvent was then removed by distillation and the keto-diester was recovered by fractional distillation. The product, dimethyl 4-oxoheptanedioate was characterized by a melting point of 49-50°C. The proton and C¹³ nuclear magnetic resonance spectra were consistent with the assigned structure.

## Claims

1. Process for the preparation of a keto-diester of formula:- 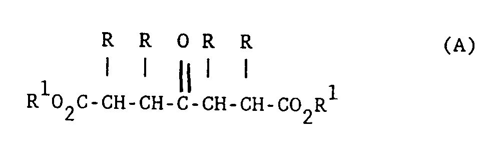 wherein each R independently represents a hydrogen atom or a methyl group, and each R¹ represents an alkyl group of up to 4 carbon atoms, which comprises reacting formaldehyde with an alkyl ester of a 2-alkenoic acid of the formula: 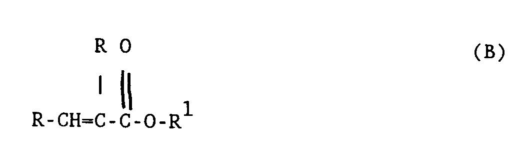 wherein R and R¹ have the same meanings as for formula (A), the reaction being carried out in the liquid phase and in the presence, as catalyst, of a thiazolium salt.

2. Process as claimed in claim 1 wherein formaldehyde is reacted with a lower alkyl ester of acrylic or methacrylic acid.

## Patentansprüche

1. Verfahren zur Herstellung von Ketodiestern der Formel: 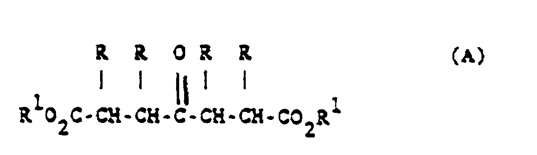 worin jedes R unabhängig voneinander für ein Wasserstofffatom oder eine Methylgruppe steht und jedes R¹ für eine Alkylgruppe von bis zu 4 Kohlenstoffatomen steht, welches die Umsetzung von Formaldehyd mit einem Alkylester von 2-Alkensäure der Formel: 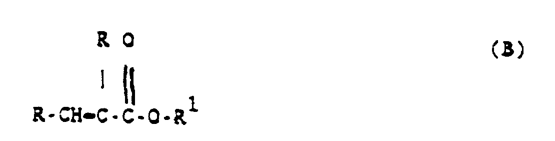 umfaßt, vorin R und R¹ dieselbe Bedeutung haben wie für Formel (A), wobei die Reaktion in der flüssigen Phase und in Gegenwart eines Thiazoliumsalzes, als Katalysator, durchgeführt wird.

2. Verfahren wie in Anspruch 1 beansprucht, worin Formaldehyd mit einem Niederalkylester der Acryl- oder Methacrylsäure umgesetzt wird.

## Revendications

1. Procédé de préparation d'un céto-diester de formule : 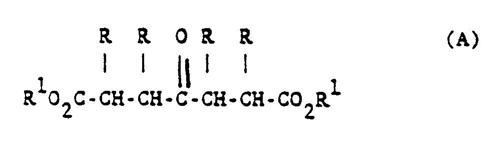 dans laquelle chaque R représente indépendamment un atome d'hydrogène ou un groupe méthyle, et chaque R¹ représente un groupe alkyle comportant au plus 4 atomes de carbone, qui comprend la réaction de formaldéhyde avec un ester alkylique d'un acide 2-alcénoïque de formule : 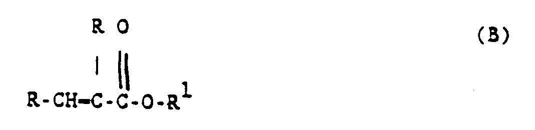 dans laquelle R et R¹ possèdent les mêmes significations que dans la formule (A), la réaction étant réalisée en phase liquide et en présence, comme catalyseur, d'un sel de thiazolium.

2. Procédé selon la revendication 1, dans lequel le formaldéhyde est mis à réagir avec un ester alkylique inférieur d'acide acrylique ou méthacrylique.
